# EUROPEAN PATENT APPLICATION

(11) **EP 4 420 714 A1**
(43) Date of publication of application: **28.08.2024**
(21) Application number: 23158332.9
(22) Date of filing: 23.02.2023
(51) Int. Cl.: A61N 1/05, A61N 1/36, A61N 1/378

(54) **AUTOMATIC RANDOM TONIC AND BURST STIMULATION DELIVERY**

(71) Applicant: Man & Science SA, 1435 Mont-St-Guibert (BE)
(72) Inventor: MEVEL, Hervé, 1450 Chastre (BE); VAN BUYTEN, Jean-Pierre, 9250 Waasmunster (BE)
(74) Representative: Patentanwälte Olbricht Buchhold Keulertz

(57) **Abstract**

The invention disclosed herein relates to a neurostimulation system for treating head and facial pain, the system comprising: an external unit configured for a location external to a subject's body; and an implant unit configured for implantation inside the subject's body; wherein the external unit comprises: a primary coil configured to transmit a stimulation signal to the implant unit; and a power source; wherein the implant unit comprises: a plurality of electrodes; and a secondary coil in electrical communication with the plurality of electrodes, wherein the secondary coil is configured to receive the stimulation signal from the primary coil; and wherein the system is configured, for each stimulation event of a series of stimulation events, to select a sub-group of electrodes from the plurality of electrodes for stimulation delivery, and/or to select a set of stimulation parameter values for stimulation delivery. According to another aspect, the invention relates to a method for selecting a stimulation delivery pattern for an implant unit of said neurostimulation system.

## Description

### Technical field

The disclosed subject matter described hereafter refers to a system and a method for electrical nerve stimulation.

### Background

Neural modulation, i.e. electrical stimulation of nerves, presents the opportunity to treat many physiological conditions and disorders by interacting with the body's own natural neural processes. Neural stimulation includes inhibition (e.g. blockage), modulation, modification, regulation, or therapeutic alteration of activity, electrical or chemical, in the central, peripheral, or autonomic nervous system.

By modulating the activity of the nervous system, for example through the stimulation of nerves or the blockage of nerve signals, several different goals may be achieved. Motor neurons may be stimulated at appropriate times to cause muscle contractions. Sensory neurons may be blocked, for instance to relieve pain, or stimulated, for instance to provide a signal to a subject. In other examples, modulation of the autonomic nervous system may be used to adjust various involuntary physiological parameters, such as heart rate and blood pressure. Neural modulation may provide the opportunity to treat several diseases or physiological conditions, a few examples of which are described in detail below.

For the purpose of this disclosure, the terms "stimulation" and "modulation" are used synonymously, so long as the usage of one of those expressions in a specific context does not imply otherwise. Likewise, the expressions "device" and "unit" are used synonymously, as are the expressions "coil" and "antenna".

Typically, neural stimulators deliver therapy in the form of electrical pulses and include one or more electrode in a proximity of the target location, such as a specific nerve or section thereof. Electrical stimulation is adjustable through various parameters, such as the polarity of electrode(s), voltage, current amplitudes, pulse frequency, pulse width, configuration and selection of electrodes and others, as these define the electrical stimulation therapy to be delivered to the user in need of therapy. Such parameters may be preprogrammed or programmable to deliver the desired stimulation and end result desired from the stimulation therapy.

Among the conditions to which neural stimulation may be applied, is the occurrence of migraine headaches. Conventional treatments typically involve the use of analgesics of varying strengths. However, due to neural involvement in the sensation of pain, methods and apparatuses aimed at neural stimulation may offer a different solution. Pain sensation in the head is transmitted to the brain via afferent or sensory neurons. Such neurons may include the greater occipital nerve, the lesser occipital nerve, the third occipital nerve and the trigeminal nerve. When a subject experiences head and facial pain, such as during a migraine headache, the inhibition of these nerves may serve to decrease or eliminate the sensation of pain.

Neural stimulation may also be an effective solution to other conditions, for example, cluster headaches, or even sleep disordered breathing and hypertension. The foregoing are just a few examples of conditions to which neuromodulation may be of benefit, however embodiments of the invention described hereafter are not necessarily limited to treating only the above-described conditions.

### Prior art

The solutions known from the prior art have several disadvantages. The use of a plurality of electrodes in order to cover a greater area of stimulation is well established in the prior art. However, if the same electrodes are repeatedly used for stimulation over and over again, a habituation effect due to tissue fatigue can occur. This means that the stimulated tissue becomes less responsive to stimulation. To balance this effect, stimulation intensity may be increased, which can, however, to sever discomfort within the patient.

In order to avoid habituation, it is known to alter the electrodes after a defined number of stimulations. This process can however become very time-consuming and often requires a high number of computing capacity, since pre-programming a neurostimulator having several stimulation electrodes can be very complex. For example, an electrode arrangement of only eight electrodes already has 1372 possible combinations of configurations of 2, 3 or 4 electrodes.

### Technical Problem

The technical problem of the invention is to respond to the disadvantages of the prior art by providing a neurostimulation system that reduces the risk of tissue fatigue or habituation after a series of stimulation events and that at the same time reduces computational requirements.

### Summary of the invention

Main features of the invention are specified by claim 1. Special embodiments or beneficial variants of the invention are presented in claims 2-11 Another aspect of the invention is specified by claim 12, with beneficial aspects presented in claims 13-15.

In accordance with this disclosure, a neurostimulation system for treating head and facial pain is provided, the system comprising: an external unit configured for a location external to a subject's body, and an implant unit configured for implantation inside the subject's body, wherein the external unit comprises: a primary coil configured to transmit a stimulation signal to the implant unit, and a power source, and wherein the implant unit comprises: a plurality of electrodes configured for neuromodulation, and a secondary or coil in electrical communication with the plurality of electrodes, wherein the secondary antenna is configured to receive the stimulation signal from the primary antenna. The system is configured in such a way that, for each stimulation event of a series of stimulation events, a sub-group of electrodes is selected from the plurality of electrodes for stimulation delivery, and/or to select a set of stimulation parameter values for stimulation delivery.

With a system as described herein, it is possible to automatically and randomly select an electrode combination, i.e. a subgroup of electrodes, for each particular stimulation pulse. This way, habituation of the patient can actively be avoided, since changing the geometry of active electrodes for each stimulation event prevents tissue fatigue. The electrodes described herein are configured to generate an electric field sufficient to modulate the terminal fibers of a nerve, when placed in the vicinity thereof.

Furthermore, the system presented above may lead to an optimization of the charge injections into a target nerve as it is impossible to define a therapy program that uses all possible combinations (or sub-groups) of electrodes.

Likewise, if a set of stimulation parameter values for stimulation delivery is selected for each stimulation event in addition to or instead of the subgroup of electrodes, habituation can be avoided, since each stimulation differs from the preceding stimulation event by one or more of a group of parameters, e.g. stimulation pulse type, current intensity, pulse width etc.

The combination of any given sub-group of electrodes with any given set of stimulation parameter values may be referred to as stimulation pattern. That means that a specific stimulation pattern comprises a selected of a sub-group of electrodes and a selected set of stimulation parameter values. The duration of one stimulation pattern may preferably be within a range spanning 0,1 s to 5 s before the next stimulation pattern begins.

The sub-group of electrodes comprises a specific electrode configuration or selection, i.e. a specific set of electrodes being active in a given stimulation event. The plurality of electrodes of the implant unit may comprise at least two and up to *n* electrodes. Likewise, each sub-group of electrodes selected from those *n* electrodes may comprise between two and *n* electrodes, wherein the number of electrodes selected for each sub-group will predominantly be fewer than *n* electrodes, for example n/2 electrodes.

Furthermore, the mode of selection of sub-groups of electrodes and/or of stimulation parameter values may be chosen from the group of selection modes comprising:
- a manual mode;
- a manual and pseudo-random mode:
- a manual and random mode;
- an extended manual and random mode;
- an automatic mode;
- an automatic and pseudo-random mode;
- an automatic and random mode;
- an automatic manual and random mode;
- an automatic mode.

In the manual mode, a physician or the patient himself or another person (collectively also referred to as "controller") may define a number of electrode patterns or sub-groups of electrodes P1 to Px. Subsequently, the stimulation therapy will then be delivered following the sequence P1, P2,..., Px, P1, P2,..., Px until the therapy session ends.

In the "manual and pseudo-random" mode, it is guaranteed that all patterns P1 to Px as set by a controller are used once before a new iteration of the pre-set patterns begins. However, for each iteration of patterns, the order in which the patterns occur will be chosen randomly. For example, for a group of four patterns P1 - P4 set by a controller, the order of the first iteration might be P2, P1, P3, P4. The next iteration may be P4, P3, P2, P1, and so on. Crucially, each iteration of patterns will comprise a randomly chosen order. As is the case in the manual mode, the number of patterns set for the "manual and pseudo-random" mode is in theory not limited.

In the "manual and random" mode, a controller again defines x patterns (P1 - Px). Each pattern will be randomly selected based on its previous occurrence. This algorithm seeks to select all patterns P1 - Px with the same occurrence over a defined time period. Furthermore, the algorithm is constantly updated based on the ratio of selection of each pattern compared to all other patterns (the more often a pattern has been selected, the less likely it becomes that this pattern will be selected for the next stimulation delivery instance).

The "extended manual and random" mode is similar to or based on the modes "manual and pseudo-random" or "manual and random". However, with the "extended manual and random", pattern pulse width and/or pattern frequency may also be defined randomly within safety limits set by the controller. Said limits may for example be 100 µs - 1000 µs for the pattern pulse width and 20 Hz - 100 Hz for pattern frequency.

The automatic mode may be described as follows: First, a controller defines a number of sets of stimulation parameter values to be selected for each stimulation event (S1, S2, ...Sx). The stimulation parameter values may for example comprise stimulation pulse type, a current intensity, a pulse width, a pulse frequency, a pulse duration, and/or a duty cycle. Then, for each set of stimulation parameter values, he defines one of the following:
a) no selection of a sub-group of electrodes, i.e. no electrode restriction; or
b) only specific electrodes may be actively used; or
c) some electrodes are removed from selection.
So, the neurostimulator selects a first set of stimulation parameter values S1 and randomly assigns electrodes for stimulation delivery selected from a), b) or c) as defined above. Then the neurostimulator selects the next set S2 and again assigns electrodes for stimulation delivery selected from a), b) or c). This process may be repeated for a defined time period. In other words, as opposed to the manual modes, where the patterns P1 - Px (which each comprise a specific set of stimulation parameter values and a specific sub-group of electrodes) are manually defined by the controller, in the automatic mode each pattern is automatically generated, i.e. the sets of parameter values (S1 - Sx) are defined by the controller and to each set a sub-group of electrodes is automatically assigned to generate the stimulation patterns P1 - Px.

The automatic and pseudo-random mode is based on the above described automatic mode combined with a random selection of delivery order of each of the sets P1 to Px (similar to the manual and pseudo-random mode).

The automatic and random mode is also based on the described automatic mode. However, after a defined time period, all patterns P1 - Px should have been delivered randomly but statistically equal (similar to the manual and random mode).

All modes may in common that the sub-group selected for each stimulation event may be different from the sub-group of electrodes selected for its preceding stimulation event. In addition to the above, the set of stimulation parameter values selected for each respective stimulation event may be different from the set of stimulation parameter values selected for its preceding stimulation event. This means that for each stimulation event, the stimulation pattern may be different from the pattern of the preceding event. This way, habituation effects due to tissue fatigue can easily be avoided, because for every stimulation event, different tissue regions will be stimulated different parameters.

Furthermore, each electrode of the plurality of electrodes may be in a state selected from the group of states comprising:
- an unconnected state, meaning that the electrode is not referenced to a specific potential;
- an anode state, meaning that it is connected to an electrical charge source which can be a voltage source or a current source;
- a cathode state; meaning that it is connected to an electrical charge sink which can be a voltage source or a current source;
- a reference state, meaning that the electrode is directly connected to a specific voltage potential; and
- a recovery state, which corresponds to a period where the charges that have been delivered are recovered by the stimulator.

The recovery state may also be explained as follows: If the system is delivering the stimulation by use of a current source, the stimulation phase may be described as I . W = Q (where I (mA) = current; W (s) = current pulse duration; Q (µC) = charge = I × t (s)). In order to go balance the charges and to go back to the initial state, the recovery phase is described as (I/x) · (x · t). Charge balance can be achieved with an active reverse stimulation, for example, with an intensity that is 10 times lower than the stimulation intensity and that has a duration = 10 · W. So the total charge delivered is equal to the total charge recovered.

Preferably, at least one of the electrodes selected for a sub-group for stimulation is a cathode and at least one other electrode selected for a sub-group is an anode. According to one embodiment, the plurality of electrodes may comprise at least two electrodes, wherein each of the plurality of electrodes may be made from a biocompatible and electrically conductive material.

The stimulation parameters for which the set of stimulation parameters values are selected and which together with the subgroup of electrodes compose the stimulation pattern, may comprise:
- a stimulation pulse type;
- a current intensity or a voltage amplitude;
- a pulse width;
- a pulse frequency;
- a duty cycle.
The stimulation pulse type defines the waveform of the stimulation delivered during a stimulation event. For example, the stimulation pulse type may comprise a tonic waveform or a biphasic waveform. A tonic stimulation pulse type comprises the following: a first stimulation pulse, followed by a passive recovery period, (optionally) followed by an active recovery period (i.e. a period where anode(s) become cathode(s) and cathode(s) become anode(s) in order to perform a charge balance), after which the process may repeat. In other words, each stimulation pulse is followed by a recovery period, wherein the recovery period may be composed of an active recovery, an passive recovery or a combination of both. The passive recovery period may also be a pseudo passive recovery period: The pseudo passive recovery period may start passively but can be terminated actively. During each recovery period, charge balances can be performed (electrical charges that have been given off need to be balanced out). It may also be possible to perform the active recovery phase before the stimulation phase, increasing the dynamic up to two-fold. In general, a tonic stimulation pulse is known as a stimulation pulse followed by a passive recovery. Therefore, it may be beneficial to have an active recovery phase or another method to discharge the safety serial capacitors in case those capacitors were implemented in the electronic circuit. This lowers the risk of damaging the electrodes (DC currents may lead to electrode material released into the body tissue). It also lowers the risk of blocking capacitors, since those capacitors would be fully charged if not actively discharged.

The biphasic waveform is characterized by stimulation divided into two phases of opposite polarity. The first phase is identical to a monophasic waveform (although usually of a shorter duration) and abruptly truncated. The second phase comprises an electrical discharge in the opposite polarity. Preferably, the stimulation may be performed at high intensity and with short duration, and with an active recovery at low intensity (below the stimulation threshold) but for a longer period of time. A biphasic current stimulation will perform an automatic charge balance if both pulse integrals are identical (i.e. if they have same 'intensity × duration' value). In that case, no additional recovery period is needed.

The current intensity may preferably be chosen from a range spanning 0.1 V to 10 V (with increments of 0.1 V) or 0.1 mA to 10 mA (with increments of 0.1 mA). The pulse width may preferably be chosen from a range spanning 100 µs to 1000 µs. The pulse frequency may preferably be chosen from a range spanning 20 Hz to 100 Hz.

The duty cycle may preferably be chosen from a range spanning from 0.1 seconds to 5 minutes. Furthermore, the duty cycle may consist of an "ON" period, during which a stimulation therapy is delivered, and "OFF" period, during which no stimulation therapy is delivered. This way, the implant unit may require less energy/power. The duty cycle is used to increase the autonomy of the system. Instead of putting the stimulator in low-power mode with no therapy delivery, the external unit is put in low-power mode (i.e. no power is sent to the implant unit over the inductive link).

Preferably, energy delivered by the implant unit may be drawn from a fix or programmable current source, or from fix or programmable voltage source, or from a combination of a current source and a voltage source, or by discharging a pre-loaded capacitor.

In addition to the above, the system may comprise at least one energy source for each stimulation pulse or series of stimulation pulses, the energy source being selected from the group comprising:
- a current source;
- a voltage source; and
- a pre-loaded capacitor.

Furthermore, the plurality of electrodes may be arranged in an electrode array. It is also possible that the plurality of electrodes may be arranged on a substrate, the substrate preferably being a lead or a paddle. The electrode paddle may comprise at least one flat substrate with the plurality of electrodes being arranged on only one side of the flat substrate (one-sided electrode paddle), or the electrode paddle may comprise at least one flat substrate with the plurality of electrodes being arranged on both sides of the flat substrate (two-sided electrode paddle). The electrode array (e.g. the lead or the paddle) may comprise a biocompatible material, such as silicone. A lead or paddle can cover a bigger tissue area, meaning that different nerves can be reached depending on which sub-groups of electrodes are used.

According to another aspect of this invention, a method for selecting a stimulation delivery pattern for an implant unit of a neurostimulation system is presented, the method comprising:
prior to each stimulation event, select a sub-group of electrodes from the plurality of electrodes for stimulation delivery, and/or select a set of stimulation parameter values for stimulation delivery, wherein the mode of selection of sub-groups of electrodes and/or of stimulation parameter values is selected from the group of selection modes comprising:
- a manual mode;
- a manual and pseudo-random mode:
- a manual and random mode;
- an extended manual and random mode;
- an automatic mode;
- an automatic and pseudo-random mode:
- an automatic and random mode;
- an automatic manual and random mode;
- an automatic mode.

It is also possible, that the sub-groups of electrodes may be selected from a predefined list of sub-groups of electrodes. In addition to the above, the stimulation parameter values are selected from a predefined range of stimulation parameter values.

The implant unit may comprise only basic circuitry to perform the desired neurostimulation tasks. However, the implant unit does not include a battery. Instead, power supply may for example be achieved via an magnetic or inductive transcutaneous radiofrequency link (RF) between the external unit and the implant unit. According to further embodiments, the implant unit may comprise, enclosed in a sealed housing, one or more of the following: a secondary coil for receiving one or more signals from the primary coil of the external unit; a flexible carrier on which the electrical components and circuitry are arranged; an AC/DC converter; a Printed Circuit Board comprising a CPU or a memory (e.g. a very low power microcontroller); and a substrate comprising a plurality of electrodes (e.g. a stimulation and or a stimulation paddle). The housing may comprise or be made of a biocompatible material, e.g. silicone. The housing may comprise hard material in order to cover and protect the electrical components sealed therein. For example, the housing may include a cover and a casing comprising titanium or ceramic.

Since the implant unit does not comprise a power source (e.g. in form of a battery), it can be kept small. Thus, it can be implanted through a short and minimally invasive procedure. In particular, a system according to this disclosure may be implanted in a one-day hospital setting that should not last more than 30 minutes. The flexible carrier may also be fabricated with a thickness suitable for implantation under a patient's skin. For example, the implant unit may have thickness of less than 4 mm, in particular of less than about 2 mm. In a preferred embodiment, the implant unit is configured for implantation through a 3 cm incision in the subject's skin at the level of the mastoid process.

The plurality of electrodes may be located alongside or on at least one substrate, e.g. a lead or a paddle. If the plurality of electrodes is arranged on a lead, the lead can also be referred to as a stimulation bridge. A stimulation bridge according to this embodiment has an elongated shape. Preferably, the length of the stimulation bridge is at least 20 times the width or diameter of the stimulation bridge. According to an even preferred embodiment, the length of the stimulation bridge is at least 50 times the width or diameter of the stimulation bridge. For example, the lead may have a diameter of 1-3 mm and a length of up to 100 mm or, in case of bilateral stimulation, a length of up to 250 mm. Once implanted, the at least one stimulation bridge may be located in such a way inside the subject's body that it is directed towards a point of about 1 to 1.5 cm above the subject's inion. The stimulation bridge may in particular be configured for implantation in such a way that at least one modulation electrode is located along the lead close to the secondary transmission unit, which may be mounted on the flexible carrier. With a stimulation bridge as described herein, it is possible to precisely innervate or stimulate a single nerve, as opposed to only being able to stimulate a greater neural area.

According to a preferred embodiment, the lead or the stimulation bridge may be configured for bilateral stimulation or for unilateral stimulation, wherein unilateral stimulation comprises a short lead and bilateral stimulation comprises a long lead. For example, it may be intended that the short lead (unilateral stimulation) is shorter than 10 cm or about 10 cm of length, whereas the long lead (bilateral stimulation) is longer than 10 cm. A stimulation bridge configured for bilateral stimulation may for example comprise a total of 16 electrodes (i.e. two distinct sections each comprising 8 modulation electrodes)

Further, bilateral stimulation requires two incisions, one small (3 cm) incision at the level of the mastoid process and a second one 1 to 1.5 cm above the inion for implantation of the stimulation bridge. However, according to a preferred embodiment, only one channel will be required for both forms of stimulation (unilateral and bilateral). With a stimulation bridge having a lead that is longer than 10 cm, i.e. that is configured for bilateral stimulation, it is possible to stimulate a larger neural area of the subject with only to minor incisions. Thus, the medical procedure of implanting the implant unit can be kept relatively short and still allow for a very efficient neural stimulation.

As part of a preferred embodiment, the implant unit may comprise tines or spikes for fixation of the at least one lead to a tissue of the subject. This way, dislocation of the implant unit from the tissue (so-called "device migration") can be avoided, which would otherwise occur due to vibrations, neck movements of the subject or simply due to gravity. The tines can be disposed on the flexible carrier and/or on the at least one lead of the implant unit and may preferably be made from a biocompatible material. According to another embodiment, fixation of the main body of the implant unit may comprise meshes. It is also possible that RX markers are placed along the lead to verify correct placement during the surgery procedure by use of an RX machine and a screen for the lead and RX markers visualization.

In order to further prevent the implant unit from migrating, the housing may also comprise one or more suture holes. This way, the implant unit can be held in place and device migration is avoided.

Any one of the embodiments, examples or features disclosed herein may be used in combination or separately and in conjunction with any one of the aspect of the disclosed subject matter, mutatis mutandis.

### Brief description of the drawings

The accompanying drawings, which are incorporated in and constitute a part of this specification, illustrate several examples of the disclosed subject matter. The drawings show the following:
- Fig. 1: depicts a schematic illustration of an implant unit having a stimulation lead;
- Fig. 2: depicts a technical illustration of an implant unit having a stimulation lead;
- Fig. 3: depicts an explosive view of a housing of the implant unit of Fig. 2;
- Fig. 4: depicts a schematic illustration of a one-sided stimulation paddle;
- Fig. 5: depicts a schematic illustration of a two-sided stimulation paddle.

### Detailed description of the drawings

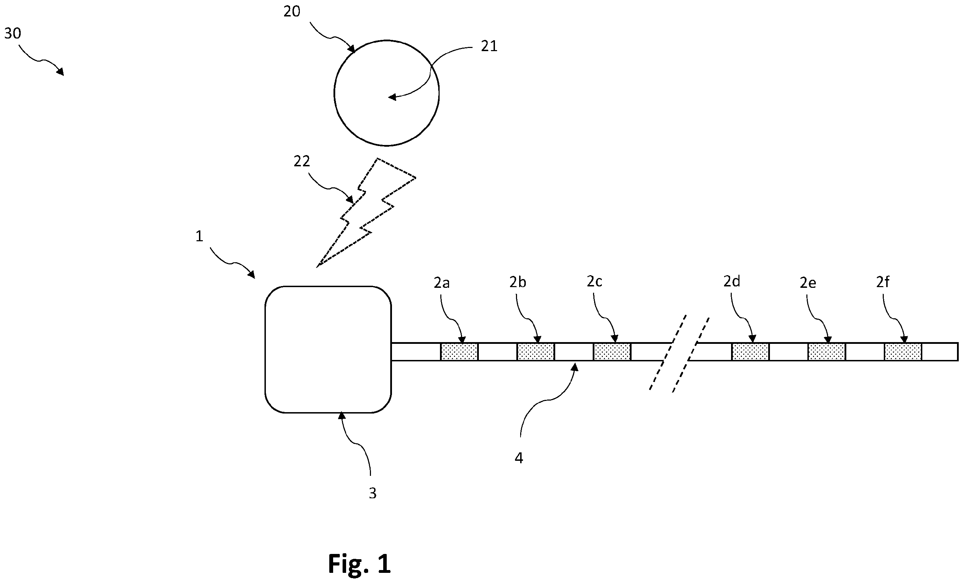

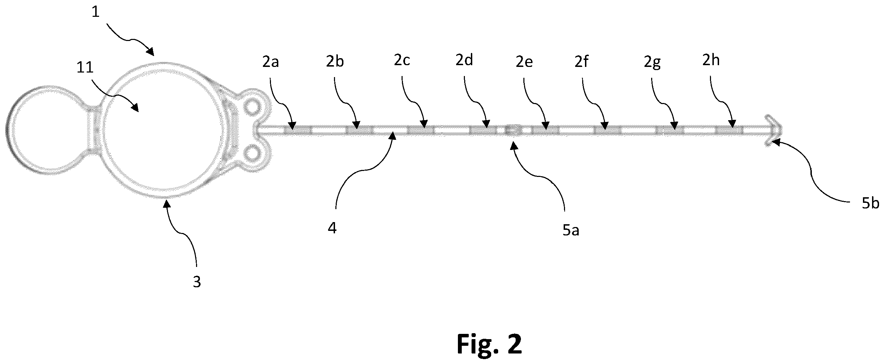

Fig. 1 and Fig. 2 depict an implant unit 1 including a housing 3 and a stimulation lead 3. The stimulation lead 3 comprises a plurality of electrodes 2a-k. Fig. 1 shows a schematic illustration and Fig. 2 shows a technical illustration of an exemplary implant unit 1. According to the example shown in Fig. 1 and Fig. 2, the housing 3 is connected to a stimulation lead 3 which a plurality of electrodes 2a-k is arranged. The lead 3 shown in Fig. 2 may be approximately 10 cm long and as well as and eight electrodes 2a-k ("octopolar lead"), which are basically evenly spaced apart along the lead 3. Due its length, the stimulation lead 3 shown in Fig. 1 and Fig. 2 can be used for bilateral stimulation, i.e. stimulation of a nerve from more than one sides. It is therefore possible to stimulate a large neural area of the subject with only few minor incisions. Thus, the medical procedure of implanting the implant unit 1 in the subject can be kept short and still allow for a very efficient neural stimulation.

According to the example shown in Fig. 2, the stimulation lead 3 may comprise at least one tine 5a,b or pair of tines 5a,b for fixation of the lead 3 to a tissue of the subject. This way, dislocation of the implant unit 1 from the tissue can be avoided, which would otherwise occur due to neck movements of the subject. The tines 5a,b can be disposed on the flexible silicone tube of the lead 4 and may preferably be made from a biocompatible material.

As disclosed herein, a group of electrodes 2a-k of the plurality of electrodes 2a-k can be randomly selected as part of a stimulation pattern for each stimulation event. For example, for one stimulation event, electrodes 2a, 2d and 2e may be selected to generate an electrical field, whereas for the next stimulation event the electric field may be generated by electrodes 2b, 2c, 2d, and 2f, and so on.

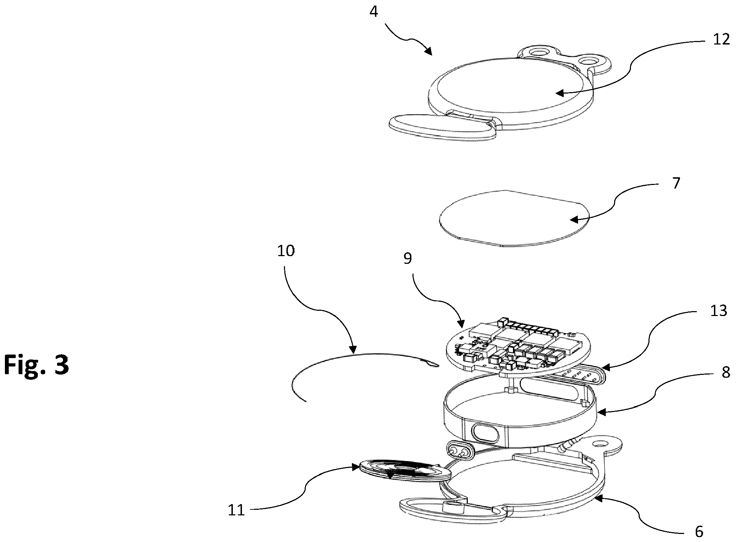

Fig. 3. depicts an explosive view of the housing 4 of the implant unit 1 shown in Fig. 1 and Fig. 2. Preferably, the housing 4 shown in Fig. 3 comprises a bottom part 6, a casing 8 in the form of a concentric cladding, and a top part 12. The three parts can be clipped, screw, clamped and/or glued together to form a sealed housing 4. The housing 4 may contain a PCB 9 with a cover element 7 disposed on in it to protect the electronic components arranged on the PCB 9, and a secondary coil 11 functioning as transceiving antenna for receiving electromagnetic or radio signals from an external primary antenna. Among other electrical components, the PCB 9 may include a CPU. Furthermore, the housing 4 can be electronically connected with the stimulation lead 3 via the means of lead contacts 13. Notably, however, the housing 4 of the implant does not contain a battery, making it possible for the implant unit 1 to be relatively small (25 mm diameter). The housing 4 may further be made of a biocompatible material, e.g. silicone. The top part 12 and the cover 7 may comprise a hard material in order to cover and protect the electrical components sealed therein. For example, the top part 12 and the cover 7 may comprise titanium.

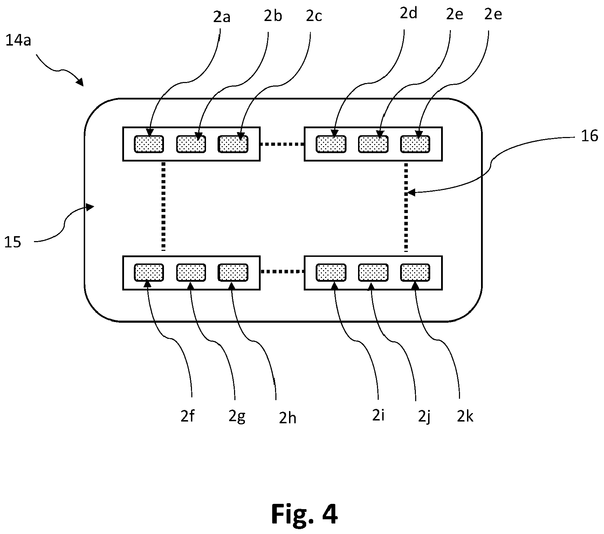

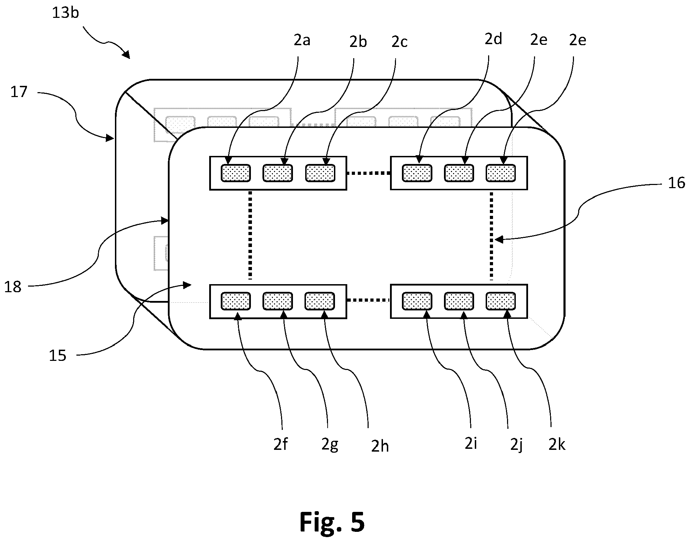

Fig. 4 and Fig. 5 depict schematic illustrations of a one-sided electrode paddle 14a and a two-sided electrode paddle 14b, respectively. According to such an embodiment, the plurality of electrodes 2a-k may be arranged in an array, i.e. the plurality of electrodes 2a-k is arranged on a flexible, biodegradable substrate 15. The electrodes 2a-k may further be connected to each other through electrical wiring 16. According to the embodiment shown in Fig. 5, the electrodes 2a-k may be arranged on two opposite sides of the substrate 15, wherein the paddle comprises a bottom surface 17 and a top surface 18.

### List of reference numerals

- 1: Implant unit
- 2a-k: Electrodes
- 3: Lead
- 4: Housing
- 5a,b: Tines
- 6: Bottom part
- 7: Cover
- 8: Casing
- 9: Printed Circuit Board
- 10: BLE antenna
- 11: Secondary coil
- 12: Top part
- 13: Lead
- 14a: One-sided electrode paddle
- 14b: Two-sided electrode paddle
- 15: Substrate
- 16: Wiring
- 17: Bottom surface
- 18: Top surface

- 20: External unit
- 21: Primary coil
- 22: Inductive transcutaneous radiofrequency link

- 30: System

## Claims

1. A neurostimulation system (30) for treating head and facial pain, the system (30) comprising:
an external unit (20) configured for a location external to a subject's body;
and an implant unit (1) configured for implantation inside the subject's body;
wherein the external unit (20) comprises:
a primary coil (21) configured to transmit a stimulation signal to the implant unit (1);
and a power source;
wherein the implant unit (1) comprises:
a plurality of electrodes (2a-k);
and a secondary coil in electrical communication with the plurality of electrodes (2a-k), wherein the secondary coil is configured to receive the stimulation signal from the primary coil (21);
and wherein the system (30) is configured, for each stimulation event of a series of stimulation events, to select a sub-group of electrodes (2a-k) from the plurality of electrodes (2a-k) for stimulation delivery, and/or to select a set of stimulation parameter values for stimulation delivery.

2. The neurostimulation system (30) of claim 1, **characterized in that** the mode of selection of sub-groups of electrodes (2a-k) and/or of stimulation parameter values is selected from the group of selection modes comprising:
- a manual mode;
- a manual and pseudo-random mode:
- a manual and random mode;
- an extended manual and random mode;
- an automatic mode;
- an automatic and pseudo-random mode:
- an automatic and random mode;
- an automatic manual and random mode;
- an automatic mode.

3. The neurostimulation system (30) of any of the preceding claims, **characterized in that** the sub-group of electrodes (2a-k) selected for each respective stimulation event is different from the sub-group of electrodes (2a-k) selected for its preceding stimulation event.

4. The neurostimulation system (30) of any of the preceding claims, **characterized in that** the set of stimulation parameter values selected for each respective stimulation event is different from the set of stimulation parameter values selected for its preceding stimulation event.

5. The neurostimulation system (30) of any of the preceding claims, **characterized in that** each electrode of the plurality of electrodes (2a-k) is in a state selected from the group of states comprising:
- an unconnected state;
- an anode state;
- a cathode state;
- a reference state; and
- a recovery state.

6. The neurostimulation system (30) of any of the preceding claims, **characterized in that** the plurality of electrodes (2a-k) comprises at least two electrodes (2a-k), wherein each of the plurality of electrodes (2a-k) is made from a biocompatible and electrically conductive material.

7. The neurostimulation system (30) of any of the preceding claims, **characterized in that** the stimulation parameters comprise
- a stimulation pulse type;
- a current intensity;
- a pulse width;
- a pulse frequency;
- a duty cycle.

8. The neurostimulation system (30) of claim 7, **characterized in that** the stimulation pulse type is preferably a tonic waveform or a biphasic waveform.

9. The neurostimulation system (30) of any of the preceding claims, **characterized in that** the system (30) comprises at least one energy source for each stimulation pulse or series of stimulation pulses, the energy source being selected from the group comprising:
- a current source;
- a voltage source; and
- a pre-loaded capacitor.

10. The neurostimulation system (30) of any of the preceding claims, **characterized in that** the plurality of electrodes (2a-k) is arranged in an electrode array.

11. The neurostimulation system (30) of any of the preceding claims, **characterized in that** the plurality of electrodes (2a-k) is arranged on a substrate (15), the substrate (15) preferably being a lead (3) or a paddle (14a, 14b).

12. A method for selecting a stimulation delivery pattern for an implant unit (1) of a neurostimulation system (30) as claimed in any of the preceding claims, the method comprising:
prior to each stimulation event, select a sub-group of electrodes (2a-k) from the plurality of electrodes (2a-k) for stimulation delivery, and/or select a set of stimulation parameter values for stimulation delivery,
wherein the mode of selection of sub-groups of electrodes (2a-k) and/or of stimulation parameter values is selected from the group of selection modes comprising:
- a manual mode;
- a manual and pseudo-random mode:
- a manual and random mode;
- an extended manual and random mode;
- an automatic mode;
- an automatic and pseudo-random mode:
- an automatic and random mode;
- an automatic manual and random mode;
- an automatic mode.

13. The method of claim 12, **characterized in that** each stimulation event is followed and/or preceded by a recovery period, wherein the recovery period is one of the following:
- a passive recovery period;
- an active recovery period;
- a pseudo passive recovery period.

14. The method of one of claims 12 or 13, **characterized in that** the sub-groups of electrodes (2a-k) are selected from a predefined list of sub-groups of electrodes (2a-k).

15. The method of one of claims 12 to 14, **characterized in that** the stimulation parameter values are selected from a predefined range of stimulation parameter values.
